# EUROPEAN PATENT SPECIFICATION

(11) **EP 1 389 089 B1**
(45) Date of publication and mention of the grant of the patent: **26.08.2009**
(21) Application number: 02737899.1
(22) Date of filing: 26.03.2002
(51) Int. Cl.: A61K 9/127, A61K 9/107

(54) **METHOD AND COMPOSITION FOR SOLUBILISING A BIOLOGICALLY ACTIVE COMPOUND WITH LOW WATER SOLUBILITY**
VERFAHREN UND ZUSAMMENSETZUNG ZUR SOLUBILISIERUNG EINER BIOLOGISCH WIRKSAMEN VERBINDUNG MIT GERINGER WASSERLÖSLICHKEIT
METHODE ET COMPOSITION PERMETTANT DE SOLUBILISER UN COMPOSE BIOLOGIQUEMENT ACTIF A FAIBLE SOLUBILITE DANS L'EAU

(30) Priority: 27.03.2001 EP 01302841
(43) Date of publication of application: 18.02.2004
(73) Proprietor: PHARES PHARMACEUTICAL RESEARCH N.V., Curacao, Netherlands Antilles (AN)
(72) Inventor: LEIGH, Steve, 1000CX Amsterdam (NL); LEIGH, Mathew Louis Steven, CH-4052 Basel (CH); VAN HOOGEVEST, Peter, CH-4416 Bubendorf (CH); TIEMESSEN, Henricus, D-79576 Weil am Rhein (DE)
(74) Representative: Hasler, Erich
(86) International application number: PCT/EP2002/003371
(87) International publication number: WO 2002/080883

(56) References cited:
- EP-A- 0 331 755
- EP-A- 0 974 364
- WO-A-00/16770
- WO-A-88/06442

## Description

The present invention relates to a kit and a method for solubilising a biologically active compound with low water solubility as defined in the claims.

In this specification, the following definitions apply:
"Lipid" refers to membrane lipids which include phospholipids, glycolipids, ceramides, gangliosides and cerebrosides. The term as used herein refers to lipids of one single type only as well as to mixtures thereof, including enzyme modified versions.
"Lipid suspension" refers to an aqueous dispersion of discrete lipid particles comprising at least one membrane lipid as the major component or constituent.
"Compounds" are biologically active substances that have a physiological and/or pharmacological effect in a living organism.
"Container" means an ampoule or vial with rubber stopper and cap, single or double chamber syringe, infusion bag or bottle made from polymeric materials or glass, suitable for parenteral administration. It also includes any vessel for holding liquids.
"Low water solubility" means any compound that requires more than 30 parts of water to dissolve 1 part of the compound. It spans the definitions between sparingly soluble (from 30 to 100) to very slightly soluble (from 1000 to 10'000) as defined in USP 24. The description includes lipophilic and hydrophobic compounds.
"Molecular associates" are complexes formed with the compound and the lipids whereby the molecules are homogeneously dispersed or solubilised in the lipid.
"molecular association " between compound and lipid molecules is achieved if not more than 20% of un-associated test material is retained on a 200 nm polycarbonate membrane filter after filtration of the lipid mixture containing the test material with distilled water.
"Loading" means incorporating or transfering compounds into lipid particles to form molecular associates.

A major problem in delivering biologically active compounds concerns poor aqueous solubility of the compounds. The problem applies in particular to lipophilic compounds that are administered by parenteral or intravenous injection. Because of its low solubility, the compound may precipitate before or after an iv injection or infusion and cause capillary blockage. As a result of precipitation and aggregation, sufficient concentrations of the drug may not be available to bind on to lipoproteins in order to be transported to target receptors and organs. Therefore, it is necessary to solubilise lipophilic compounds to elicit the required therapeutic effects.

In the prior art, ethanol and aqueous solutions of detergents like Cremophor EL® or polysorbate 80 are commonly used to solubilise lipophilic compounds. Alternatively, they may be complexed with hydroxypropyl-beta-cyclodextrins or dissolved in an oil/water emulsion system. However, precipitation of the drug on dilution of the organic solvent is a problem and anaphylaxis following injection with Cremophore EL is not an unknown problem. Oil-in-water emulsions are restricted to compounds with sufficient oil solubility. Furthermore, the compounds may accelerate physical instability of oil-in-water emulsions and some may not be stable to withstand heat sterilisation or storage. Liposomes comprising phospholipid vesicles are sometimes used to deliver poorly water soluble compounds such as amphotericin. The low toxicity and high tolerability of phospholipids make liposomes an attractive vehicle for delivering lipophilic compounds. However, a wider commercial use of lipid particles containing lipophilic drugs requires problematic and expensive manufacturing procedures to associate the lipophilic drug with the lipids. Examples of such procedures include high shear and/or high pressure homogenisation, controlled organic solvent dilution, cross flow filtration to produce aqueous liposomal suspensions containing the drug ( see e.g. Isele, U.; Van Hoogevest, P.; Hilfiker, R.; Capraro, H-G.; Schieweck, K. and Leuenberger, H., Large-Scale Production of Liposomes Containing Monomeric Zinc Phthalocyanine by Controlled Dilution of Organic Solvents, J. Pharm. Sci. (1994), 83,1608-1616.). Even if production problems can be overcome, the majority of drugs and phospholipid are chemically or physically unstable in water in the solubilised state and will need to be lyophilised for storage. The problem here is that lyophilised liposomes may require expensive synthetic or semi-synthetic lipids with high phase transition temperatures to maintain physical stability on reconstitution, further adding to already high costs.

In numerous prior art references the use of phospholipids to solubilise compounds with low water solubility is described:
WO 98/58629 describes lipid compositions comprising at least one monoacyl lipid, e.g. monoacyl phospholipid and mixtures of mono acyl and diacyl phospholipids that are effective in carrying lipophilic compounds in molecular form. The compositions may be a waxy solid, a paste-like material or a viscous fluid suitable for filling into hard or soft gelatine capsules. There is no mention of an extemporaneous preparation of an injectable drug formulation.

The preparation of lipid-drug co-precipitates using diacyl phospholipids to increase the dissolution behaviour of poorly water soluble drug solvates, and the possibility of modifying drug release from such dispersions by incorporating small amounts(<0.05%) of polyvinyl pyrollidone is described in J. Pharm. Sci. 81, 283-286 (1992). The compositions are prepared essentially by co-precipitation and result in the incorporation of lipid in the crystalline structure of the solvate. The residual solvent trapped in the solvate crystals is given as a possible reason for the improved solubility of the poorly water soluble compound. An in situ preparation of an injectable drug formulation is not described.

WO 86/05694 describes the use of non-esterified fatty acids and monoglycerides together with minor amounts of a monoacyl lipid (lyso phosphatidylcholine) to form solid particles which show improved oral absorption for various lipophilic compounds. Improved oral absorption is explained to be due to the unique properties of the mixture. An in situ preparation of an injectable drug formulation is not described.

US-A-5,091,188 discloses injectable compositions and methods for rendering insoluble or poorly soluble powders more stable, by stabilising the external surfaces with one or more layers of phospholipids to prevent the particles of drug from agglomeration during storage. The drug is not in molecular dispersion and an in situ preparation of an injectable formulation is not described.

WO 99/49846 discloses compositions and procedures that yield sub-micron and micron size stable particles of water-insoluble drugs together with phospholipids, a charged surface modifier and a block copolymer adhered to the surfaces to prevent the particles from particle growth, aggregation or flocculation in suspension. The particles are not in molecular dispersion with the lipid molecules. High shear mixing by means of multiple passes through a micro fluidiser is necessary to reduce the size of the drug particles. There is no mention of an extemporaneous preparation of an injectable drug formulation.

WO 99/65469 discloses submicron particles of water-insoluble drugs, prepared simultaneously by stabilising microparticulate suspensions of the drug with surface modifier molecules eg a phospholipid, by rapid expansion into an aqueous medium from a compressed solution of the compound and surface modifiers in a liquified gas. The particles of the drug are surface stabilised in the suspension and prevented from agglomerating. The particles are not in molecular dispersion and there is no mention of an in situ preparation of an injectable drug formulation.

EP-A-0 795 585 discloses a process for preparing finely divided suspensions of a particulate retinoid or caretinoid in a volatile, organic solvent mixed with aqueous medium in the presence of a physiologically compatible emulsifying agent. An example of the various emulsifying agents used is a hydrolysed lecithin (Emulfluid E) which contains a substantial amount of non polar oils and free fatty acids ie < 45%. The retinoid or caretinoid is not in molecular dispersion. The described compositions are not suitable for parenteral use.

EP-B-0 256 090 describes the use of a specific monoacyl lipid, i.e. lyso-phosphatidylethanolamine, alone or in combination with other diacyl phospholipids to solubilise hydrophobic materials inside small unilamellar vesicle (SUV) suspensions. There is no mention of an in situ preparation of an injectable drug formulation.

EP-B-0158 441 relates to pro-liposome compositions based on membrane lipids, to a method of making lipid vesicles by the addition of aqueous fluid to these compositions, and to aqueous dispersions of vesicles. The compositions contain water soluble, or oil-soluble biologically active compounds. They may also contain an organic solvent suitable for injection purposes, such as ethanol. An in situ preparation of an injectable drug formulation is not described.

WO 97/25977 discloses a process for extemporaneous preparation of an oil-in-water fat emulsion composition comprising a cyclosporin, a rapamycin or an ascormycin or a derivative thereof, which comprises the step of admixing to a placebo fat emulsion a concentrate comprising the active, a stabiliser (e.g. phospholipid) and an organic solvent. There is no mention of an in situ preparation of an injectable composition comprising lipid particles wherein the major component is a phospholipid containing a poorly soluble compound and the patent is explicitly on cyclosporins, rapamycins and ascormycins and their derivates only.

US-A-5,747,066 and US-A-4,158,707 describe mixed micelles for the aqueous solubilisation of active substances which are only poorly soluble or insoluble in water to obtain a solution with improved storage properties which consist of a phosphatide and a bile salt. There is no mention of an in situ preparation of an injectable drug formulation.

US-A-5,192,549 discloses a method of amphipatic drug loading into liposomes by pH gradient, whilst US-A-5,316,771 describes amphipatic drug loading into liposomes by ammonium ion gradient. US-A-5,380,531 also describes a method for an accumulation of amino acids and peptides into liposomes. These three examples are restricted to loading of preformed liposomes with compounds which are water soluble and possess a basic function which can be protonated.

In US-A-5,676,341, high drug:lipid formulations of liposomal antineoplastic agents are provided. Liposomes may be made by a process that loads the drug by an active mechanism using a transmembrane ion gradient, preferably a transmembrane pH gradient. Using this technique, trapping efficiencies approach 100%, and liposomes may be loaded with drug immediately prior to use, eliminating stability problems related to drug retention in the liposomes. Drug:lipid ratios employed are about 3-80 fold higher than for traditional liposome preparations, and the release rate of the drug from the liposomes is reduced. An assay method to determine free antineoplastic agents in a liposome preparation is also disclosed. The disclosed method is restricted to loading of ionizable antineoplastic agents and the therapeutic use of this approach is clearly intended.

EP-A-0 974 364 addresses parenteral administration of basic or acidic drugs which have high solubility at a non physiological pH in water but have a low water solubility at a physiological pH. Aqueous solutions containing the drug are mixed with a fat emulsion or liposomal dispersions. The reference is silent with respect to parenteral administration of drugs which are poorly soluble in aqueous media independent of pH considerations.

WO 88/06442 A is directed to a method for encapsulation of antineoplastic agents in liposomes. The reference teaches the encapsulation of cytostatic drugs, which are clearly water soluble. The solvents used are not hydrophilic. The loading of the liposomes is accomplished by an active mechanism using a trans-membrane ion gradient, preferably a trans-membrane pH gradient. The reference is silent with respect to parenteral administration of drugs which are poorly soluble in aqueous media independent of pH considerations.

It is apparent that there exists a practical need for an efficient method to associate lipophilic drugs with lipid particles. An object of the present invention is to provide an improved method to solubilise hydrophobic compounds as molecular associates in lipid particles wherein the major component comprises at least one membrane lipid, particularly but not exclusively intended for parenteral use. It is an aim of the invention to provide sterile compositions that may be prepared in situ, just prior to use, to avoid stability and storage problems. It is a further object of the invention to provide a method that is reproducible, commercially viable and cost effective, practical and can be validated. It is also an object that the components used are safe, readily available and can be rendered sterile. It is yet a further object that the invention may be used in medical applications, clinical research and pre- clinical screening applications, such as, i.e., in-vitro cell or in-vivo animal efficacy/toxicity studies, and for solubilising compounds in lipid carriers that may be processed further for internal and external applications.

The present invention addresses the aforementioned aspects in providing cost effective, simple compositions and a method which avoids production and stability problems. Furthermore, it allows the use of reliable and cost effective phospholipids without the need to use expensive synthetic or semi-synthetic lipids. Unexpectedly, it has been found that the present invention allows compounds which have low solubility in aqueous media to form molecular associates spontaneously and instantaneously with membrane lipid suspensions comprising discrete particles wherein the major constituent is at least one membrane lipid, when they are mixed together. Furthermore the invention allows the two components to be kept apart in separate containers until the moment of mixing. The poorly soluble compound may be dissolved in a hydrophilic medium or it may be present in a dry powder form. Preferably, the lipid suspension is transparent and the resulting. composition comprising lipid associates is particularly suitable for parenteral use but may also be applied for oral, pulmonary and topical administrations to a living organism.

The lipid suspension and the compound are prepared as separate compositions and held in two separate containers. The lipophilic compound in the first container is preferably dissolved in a hydrophilic medium or presented as a powder or lyophilisate. The lipid suspension in the second container is suitable for long term storage and can be manufactured using standard high pressure homogenisers and/or extruders. It may be sterile filtered and in some cases even heat sterilised. Shortly before administration the contents of one container is added to the other. Because of the lipophilicity of the compound and the extensive surface area presented by the discrete lipid particles, the compound partitions preferentially into the lipid and forms molecular associates. Therefore, as loading is effected instantly by means of partition and molecular association, the process may be described as Instant Partition Loading (IPL).

Surprisingly it has been found that an aqueous suspension of finely divided lipid particles, i.e. preformed particles comprising at least one membrane lipid as the major component or constituent has a much higher capacity for solubilising lipophilic compounds if the compound is added to already formed lipid particles and not dissolved in a lipid solution during formation of the particles, which is usually the case in the prior art. Thus, the invention comprises a composition for solubilising or forming molecular associates which further comprises a compound with low water solubility held in a first container either as a solution in a physiologically acceptable hydrophilic medium, e.g. ethanol, or as a powder or a lyophilisate, and a discrete suspension of at least one membrane lipid particles held in a second container, until the contents of the two containers are admixed. The lipid particles further comprise at least one membrane lipid as the major component. Optionally physiologically acceptable exipients and components such as stabilisers and preservatives may be present in one or both containers or in still a further container. In another aspect of the invention, a method of loading said lipophilic compounds into a preformed suspension of said discrete lipid particles is provided which involves mixing the contents of the two containers to form molecular associates. The resulting composition comprising molecular associates is particularly suitable for injection but may also be used for other applications. The method according to the invention is characterised by a high loading efficiency and practicality. Compared to prior art methods of sequestering lipophilic compounds in liposomes, the loading by means of partitioning and molecular association is straightforward. There is no need for pH alterations during preparation or other manipulations to be carried out to remove extraneous material. The method particularly allows the molecular association of compounds which are poorly soluble in aqueous media independent of pH consideration and condition. By the method in accordance with the invention stability and storage problems may be avoided.

The lipophilic compound is prepared either as a solution in a hydrophilic solvent or as a powder. For highly lipophilic or unstable compounds, it may be preferable to convert the crystal form to an amorphous form that is more readily soluble. This may be carried out by precipitation and/or lyophilisation or any other methods such as milling, with or without stabilisers. The lipophilic compound is held in a vial or other types of vessel either dissolved in a suitable hydrophilic medium or as a lyophilised powder, both containing optionally other excipients.

### Method

Typically, an aqueous suspension of discrete lipid particles is prepared in bulk. The lipid dispersion comprises between 0.5% w/w to 25% w/w, preferably below 20% w/w, most preferably between 5% w/w to 15% w/w, of at least one membrane lipid, preferably a phospholipid, suspended in an aqueous medium, optionally containing solvents and surfactants such as bile salts. Any production method that results in a lipid suspension with an average particle size up to 50 µm may be employed. In particularly preferred embodiments, the aqueous lipid suspension is subjected to high pressure homogenisation or extrusion in a high pressure homogenizer to obtain particles having a size of less than 1000 nm, preferably less than 300 nm, most preferably below 100 nm, with a low polydispersity index to produce a transparent or optically clear suspension. The size refers to the Z average diameter using photon correlation spectroscopy. An optically clear suspension is a desired feature in those applications where the contents of the two containers are mixed together in situ just before application e.g. injection. Therefore optically clear lipid suspensions are a highly desirable but not an essential feature of the invention for the purpose of associating lipophilic compounds for other applications, e.g. oral use. The important feature of the invention is to obtain maximum loading of lipophilic compounds into preformed suspensions of lipid particles regardless of clarity or lamellarity. The suspension may be produced in volume and held in a second container or vessel suitable for volume production and transferred into individual unit containers such as a vial. The suspension may be vesicular, non-vesicular or combinations depending on the particular route of administration, the type of lipid employed and the properties of the compound with low water solubility. The composition may be sterilised by filtration and aseptically filled into individual sterile vials fitted with suitable rubber closures. Alternatively, the vials and contents may be terminally sterilised.

The content of one of the vials or containers may be added to the other as appropriate and mixed to form molecular associates in situ, just prior to use. Alternatively for those compounds that are more stable, the prepared or fully loaded lipid suspension may be transferred to smaller containers for long term storage. Alternatively, the lipid suspension may be lyophilised or dried by any suitable method such as fluidised bed drying or spray drying. The method of loading is rapid and practical and capable of achieving association efficiency of 80%w/w or more, preferably 90%w/w or more, most preferably 99%w/w or more. The suspension of lipid particles or the lyophilised material is particularly suitable for injection as a bolus dose as such or it may be added as a concentrate to infusion fluids. It may also be used for other purposes, e.g. in a nebuliser for inhalation and for topical applications. In cases where the lipid suspension in the second container is optically clear, any unassociated material can be seen clearly in the transparent suspension in the vial and the imperfect composition can be rejected. The small particle size allows the molecular associates to pass a safety filter prior to parenteral administration.

### Optical clarity

It is a desirable feature of the invention, particularly for parenteral use, that the suspension in the vial is transparent or optically clear i.e. allows the transmission of incident light. This may be judged by visual inspection for transparency and turbidity. Preferably, the clarity is determined initially at e.g. 660 nm or the most appropriate wave length depending on the lipid concentration, using a 1cm transmission cell or cuvette. If a second determination is carried out after the loading process (IPL), the difference in the transmitted value reflects the loading efficiency. It is based on the premise that any unassociated compound that is not molecularly dispersed will precipitate out as larger particles and increase the turbidity of the initial suspension. Generally the initial suspension of lipid particles in the second container should be sufficiently clear to allow at least 40%, preferably 60%, most preferably more than 80%, of light to be transmitted. Using these initial values as benchmarks, a loaded suspension of the same lipid particles substantially free from precipitates or unassociated drug should not decrease transmission by more than 25%, preferably not more than 10%, most preferably not more than 5%. The method gives a fast and reliable means to assess the loading efficiency of the invention when the loaded suspension is required to be made up at the point of use.

For in situ preparation of injectable compositions before administration, an optically clear lipid suspension is preferred to allow visual inspection for precipitated drug particles. For most other applications such as in oral administration, optical transparency may not be an essential feature.

It is to be clearly understood that the suspension of lipid particles that are converted to molecular associates with the drug are vesicular structures. The type of lipid particle obtained depends on the combination of diacyl to monoacyl membrane lipid component and has been described in WO 98/58629 (PCT/GB98/01803).

### Lipophilic Compound

The invention is particularly suitable for solubilising poorly water soluble compounds that are administered in single doses above about 10mg and have solubilities of less than 10 mg/100 ml in deionised water at ambient temperature. It is particularly suitable for compounds that have water solubilities of less than 1 mg/100 ml and lipophilic compounds that bind onto lipoproteins. Typical examples of biologically active lipophilic compounds that have poor water solubility, include hydrophobic immunosuppressants like neutral cyclic peptides eg. cyclosporin A, tacrolimus or a macrolide, e.g. a rapamycin.

It is to be understood further that the lipophilic compound in the first container may include other excipients which are compatible with the compound and facilitate the loading of the lipids. Optionally, the excipients may be bulking agents, membrane lipids, preferably charged lipids, bile salts or salts of fatty acids included as minor components in the composition either in solution, as a co-precipitate or as a lyophilised powder.

### Lipid

The ratio of drug to lipid is typically between 1:2 to 1:200, preferably 1:5 to 1:100, most preferably 1:5 to 1:50 parts by weight.

The lipid contains at least one membrane lipid, preferably at least one phospholipid of the formula 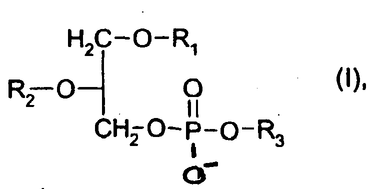 wherein
R₁ represents C₁₀-C₂₀acyl;
R₂ represents hydrogen or C₁₀-C₂₀acyl;
R₃ represents hydrogen, 2-trimethylamino-1-ethyl, 2-amino-1-ethyl, C₁-C₄alkyl, C₁-C₅alkyl substituted by carboxy, C₂-C₅alkyl substituted by carboxy and hydroxy, C₂-C₅alkyl substituted by carboxy and amino, an inositol group or a glyceryl group or a salt of such compound.

The phospholipid may be neutral or it may be charged. It may be a double chain or a single chain amphipath. Examples of neutral phospholipids with double chains are, phosphatidylcholine (PC), phosphatidylethanolamine (PE) and sphingomyelin. Examples of charged phospholipids are phosphatidic acid (PA), phosphatitdyl inositol (PI) and phosphatidylserine (PS) and phosphatidylglycerol (PG). The hydrocarbon chain can either be unsaturated or saturated and can have between 10 to 24, preferably 14 to 18 carbon atoms.

The single chain lipid is the monoacyl derivative of a neutral or charged phospholipid, but it can also be the monoacyl derivative(s) of glycolipids and sphingolipids. Deacylation may be carried out by phospholipase A2 enzyme hydrolysis or by chemical means. The hydrocarbon chain can either be unsaturated or saturated and can have between 10 to 24, preferably 14 to 18 carbon atoms. The lipids may be derived from natural plant, or animal or microbiological sources, synthesised or partially synthesised, including polyethyleneglycol (PEG) derived monoacyl phospholipids, eg. pegylated monoacyl phosphatidyl ethanolamine.

Other membrane lipids, such as glycolipids, ceramides, gangliosides and cerebrosides can be used in place of, or in partial replacement of phospholipids. The preferred membrane lipid is phosphatidylcholine (PC). Most preferred diacyl phosphatidylcholine is soy PC, followed by Egg PC, POPC, and OOPC. Most preferred monacyl counterpart is enzyme modified (Phospholipase A2) soy PC,followed by Egg PC,1 -palmitoyl PC, 1 oleoyl PC,1-stearoyl PC.

The lipid particles may comprise entirely of a diacyl lipid or a monoacyl lipid on its own or it may contain mixtures of the monoacyl and diacyl components in any combination obtained by enzyme hydrolysis, depending on the end use.

### Hydrophilic solvent

Examples of water miscible, pharmaceutically acceptable solvents are: ethanol, 96% ethanol, absolute glycerol, propylene glycol, ethyl lactate, polyethylene glycol 300, polyethylene glycol 400,1,3 butandiol, succinic acid diethyl ester, triethyl citrate, dibutyl sebacate, dimethyl acetamide, DMSO, glycerineformal, glycofurol (tetraglycol), isopropanol, lactic acid butyl ester, N-methylpyrrolidone, solketol, propylene carbonate, propylene glycol diacetate, tetrahydrofurfuryl alcohol, diethylene glycol mono ethyl ether, triacetin. The hydrophilic solvent may optionally include water. Preferably the composition should not contain more than 15% w/w of solvent in the final product after mixing the contents of the two containers, for parenteral or iv use.

### Other pharmaceutically acceptable excipients

Optionally other pharmaceutically acceptable excipients may be present, either as stabilisers or preservatives. They may be included in the second container holding the lipid suspension or in the first container holding the active compound in solution or as a lyophilised powder. Examples of stabilisers are isotonic and buffer agents, e.g. sugars and salts, or anti-oxidants, e.g alpha tocopherol acetate, ascorbyl palmitate. Examples of preservatives are anti-microbials, e.g. methyl paraben and butyl paraben. The first vial may also contain excipients which are able to form a cake upon lyophilisation, like polyethylene glycol 3000 and polyethylene glycol 4000, sugars such as mannitol and lactose and saccharose. Furthermore, the first vial may contain other excipients which improve solubility and the loading of the lipids, e.g. mono- and diacyl membrane lipids such as egg PC, soy . PC, soy PG, fatty acids and salts thereof, surfactants like polysorbate 80, poloxamer and Cremophor EL.

The following examples are given to illustrate the invention and its utility and not by way of limitation. The invention is not limited to the compounds exemplified or the scale of the production typically shown in the examples.

### Example 1

10 mg of miconazole and 1 mg of POPG are dissolved in 0.25 g of ethanol and held in a vial. 2.5 g of a 10% phospholipid (98% soya phosphatidylcholine and 2% egg PG) mixture is obtained and hydrated in 10 ml distilled water. The lipid dispersion is passed through a high pressure homogeniser (Emulsiflex C5, Avestin) to obtain an optically clear suspension. The lipid particles are smaller than 100 nm. The content of the first vial is added to the clear lipid dispersion in the second vial and shaken. The resulting dispersion of molecular associates is clear and there is no decrease in the light transmission. Over 90% of the miconozole is transferred to the lipid particles and may be confirmed by analytical filtration and HPLC analysis. The lipid suspension may be administered by inhalation using a nebuliser or it may be applied topically.

### Example 2

10 mg of triclabendazole and 5 mg of sodium oleate are dissolved in 0.5 ml ethanol in a first container with 5 ml of deionised water containing 50 mg of lactose. In place of sodium oleate a bile salt or a charged membrane lipid may be used. The resultant dispersion is immediately frozen and lyophophilised to produce a cake. To this cake 2.5 g of a 10% phospholipid dispersion (98% egg PC) produced by high pressure homogenisation is added. The appearance of the dispersion remains clear after the addition to the cake. The decrease in light transmission is less than 10%.

### Example 3

An antiviral compound C₂₃H₂₁N₅SF active with low water solubility (0.00008 g/l), melting point 179°C is associated with lipid particles as follows:
25 mg of the antiviral compound is dissolved in 975 mg of PEG400/ethanol (1:1 v/v) containing PG (2.5 mg/ml) and held in a first vial.

A 12%w/w phospholipid suspension as in Example 1 is prepared by dispersing the lipid in 2.5%w/w glycerol at room temperature, followed by passage through an Avestin high pressure homogeniser. The mean particle size of the lipid particles as measured by photon correlation spectroscopy is ca. 40 nm. This is held in a second vial.

Instantant partition loading of the antiviral compound according to the invention is performed under aseptic conditions by adding 0.4 ml of the organic drug solution in the first vial to 10 ml of lipid particles in the second vial while gently swirling the vial. The resulting lipid suspension has a particle size of 54 nm and is free from precipitated drug particles and may be injected directly or after dilution with sterile 5%w/w glucose for intravenous use. The lipid particles in this example are unilamellar vesicular structures as determined by electron microscopy. Employing different combinations of lipid, the associates formed may be non vesicular or mixed micellar structures. The resulting dispersion is physically stable for more than 24 hours. The lipid particles associated with the drug are also suitable for oral administration.

### Example 4

In this example, the first container is a vial which contains a solution of 10% w/w of cyclosporin A and 1% of egg-phosphatidylglycerol in 96 %ethanol.

A second vial contains a suspension of lipid particles comprising 10% w/w phospholipid prepared as in example 3.

The instantaneous loading of the lipid particles with cyclosporine A is performed under aseptic conditions by injecting 0.06 ml of the drug solution from the first vial into 3 ml of lipid suspension in the second vial, while gently swirling the vial. The lipid suspension has a particle size of 38 nm and does not contain precipitated drug substance. The resulting dispersion is physically stable for more than 24 hours. It may be injected either directly or after dilution with sterile 5% w/w glucose for intravenous administration. Alternatively, it may be given orally or applied topically.

### Example 5

1 g cyclosporin A and 0.1 g egg-phosphatidyl glycerol are dissolved in 3.0 ml tert-butanol, sterile, filtered through a 0.2 µm filter and the solvent removed by lyophilisation to obtain a dry dosage form with long term stability. This is held in the first container. Shortly before use (administration) 10 ml ethanol 96 % is added to the container before the contents are admixed with a second container containing a lipid suspension, as described in Example 4. This method is particularly suitable for compounds that have poor storage stability even in ethanolic solution.

### Example 6

In this example, 5 mg paclitaxel (SIGMA, 97 % pure) and 5 mg Egg phosphatidylgylcerol/ml are dissolved in absolute ethanol and held in a first container. 200 µl of the paclitaxel solution from the first container is added to a second container containing 2 ml of a lipid suspension comprising 5 %w/w phospholipid dispersion of phosphatidylcholine and PG as in the previous examples, and 1.25 %w/w glycerol and 2.5 % w/w glucose. The resulting lipid dispersion containing paclitaxel is free of precipitated drug crystals and suitable for intravenous or oral administration. For oral administration, the lipid blend may contain up to 50%w/w of monoacyl phosphatidylcholine . The decrease in light transmission is less than 5%. The resulting dispersion is physically stable for more than 24 hours. By comparison, when 200 µl of the paclitaxel solution from the first container is added to a second container containing 5% glucose without lipid particles, a coarse precipitate is formed which is not suitable for administration.

In place of paclitaxel, other taxanes may be used to form lipid associates with alternative types of lipid particles using various combinations of lipid components including monoacyl derivatives. The reason in using alternative lipid particles and lipid mixtures is to obtain molecular association of the drug molecules with the lipid components.

### Example 7

An anticancer compound C₁₀H₇N₅SBr with low water solubility (2 mg/ml), and solubility in propylene glycol < 2.5 mg/ml , in PEG 400 < 2.5 mg/ml is associated with lipid particles as follows.

100 g of the anti-cancer compound is dissolved in 1.01 of DMSO and held in a first vessel. This solution is further diluted to a concentration of 125 mg drug substance per 20 ml.

A 1 % w/w phospholipid suspension is prepared by dispersing the lipid in 50 mM Na₂HPO₄ pH 7.0 at room temperature. The lipid particles are multilamellar vesicles within the size range of 1.0 to 12 µm. This is held in a second vial.

The loading of the anticancer compound according to the invention is carried out by adding 20 ml of the organic drug solution in the first vial to 250 ml of the lipid particles suspension in the second vial while gently swirling the vial. The resulting lipid suspension is free from precipitated drug particles. After filtration/extrusion through a 0.22 µm filter and measurement by means of HPLC it is found that 100 % of the drug is able to pass the filter. By comparison without lipid in the phosphate buffer, 98 % of the drug substance is retained on the filter.

### Example 8

Alternative procedures for mixing the contents of the first and second containers.
To load the lipophilic drug into the lipid dispersion, the contents of the first vial should be mixed with the contents of the second vial (or vice versa).

| First container | Second container |
|---|---|
| Lipophilic drug in water miscible solvent | Aqueous dispersion of lipid particles |
| Lipophilic drug in dry formobtained from lyophilisation of an organic solvent | Aqueous dispersion of lipid particles |
| Lipophilic drug in dry form obtained from lyophilisation of an aqueous suspension | Aqueous dispersion of lipid particles |

## Claims

1. A process for solubilising a drug substance with low water solubility, i.e. a drug substance requiring more than 30 parts of water to dissolve 1 part of the drug substance, comprising mixing
A) a composition which is contained in a first container, comprising said drug substance dissolved in
i) at least one water miscible solvent, or
alternatively
ii) an amorphous powder or lyophilisate of the drug substance prepared from a solution of said drug substance,
with
B) a composition, which is kept in a second container, comprising
a liposomal dispersion.

2. The process according to claim 1 wherein the second container comprises a liposome dispersion which is optically clear allowing at least 40% of light to be transmitted at a wave length of 660nm using a 1 cm transmission cell or cuvette.

3. The process according to claim 1 or 2, wherein the water miscible solvent in the first container is selected from the group consisting of ethanol, 96% ethanol, absolute glycerol, propylene glycol, ethyl lactate, polyethylene glycol 300, polyethylene glycol 400, 1,3 butandiol, succinic acid diethyl ester, triethyl citrate, dibutyl sebacate, dimethyl acetamide, DMSO, glycerineformal, glycofurol (tetraglycol), isopropanol, lactic acid butyl ester, N-methylpyrrolidone, solketol, propylene carbonate, propylene glycol diacetate, tetrahydrofurfuryl alcohol, diethylene glycol mono ethyl ether, and triacetin.

4. The process according to claim 1 or 2, wherein the liposomal dispersion in the second container comprises liposomes having an average particle size which is smaller than 1000 nm.

5. The process according to claim 4, wherein the liposomal dispersion in the second container comprises liposomes having an average particle size which is smaller than 300 mn.

6. The process according to claim 1 wherein the first container comprises at least one excipient selected from the group consisting of mono- and diacyl membrane lipids such as egg phosphatidylcholine, soy phosphatidylcholine or soy phosphatidylglycerol, fatty acids and salts thereof, polysorbate 80, poloxamer and Cremophor® EL.

7. The process according to claim 1 wherein the liposomal dispersion in the second container comprises phospholipids selected from the group consisting of phosphatidylcholine, phosphatidylethanolamine, sphingomyelin, phosphatidic acid, phosphatitdyl inositol, phosphatidylserine and phosphatidylglycerol, glycolipids, gangliosides and cerebrosides.

8. The process according to claim 6 or 7 wherein the phospholipid is of the formula 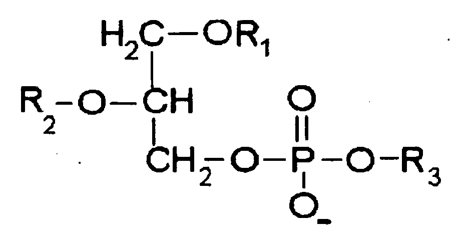
wherein R1 represents C10-C20acyl; R2 represents hydrogen or C10-C20acyl; R3 represents hydrogen, 2-trimethylamino-1-ethyl, 2-amino-1-ethyl, C1-C4alkyl, C1-C5alkyl substituted by carboxy, C2-C5alkyl substituted by carboxy and hydroxy, C2-C5alkyl substituted by carboxy and amino, an inositol group or a glyceryl group or a salt of such.

9. The process according to any one of the preceeding claims, wherein the contents of the first and second containers are mixed with each other in situ, just prior to parenteral administration, thus yielding a composition being optically clear and free from precipitates or unassociated drug substance and ready for immediate parenteral administration.

10. The process according claim 9, wherein the containers are selected from the group consisting of ampoule, vial with rubber stopper and cap, single and double chamber syringe, infusion bag or bottle, suitable for parenteral administration.

11. The process according to any one of claims 1 to 10, wherein the amorphous powder contained in the first container is prepared by precipitation and/or lyophilisation from a solution of said drug substance in a solvent.

12. A kit for solubilising a drug substance with low water solubility, comprising
i) a first container comprising
a) a drug substance with low water solubility, i.e. requiring more than 30 parts of water to dissolve 1 part of the drug substance, dissolved in
b) at least one water miscible solvent, or alternatively
c) an amorphous powder or lyophilisate of the drug substance prepared from a solution of said drug substance, and optionally
d) at least one surfactant selected from the group consisting of. phospholipids, fatty acids and salts thereof, polysorbate 80, poloxamer and Cremophor® EL, and
ii) a second container comprising a liposomal dispersion;
said kit being designed for mixing of the contents of the two containers in order to obtain an administration form of said drug substance, suitable for parenteral, oral, pulmonary and topical application to a living organism.

13. A kit according to claim 10 wherein the contents of the first and second container are aseptically filled in sterile vials or terminally sterilized.

14. Use of the process of claim 1 in in-vitro cell studies.

## Patentansprüche

1. Verfahren zur Auflösung eines Arzneimittels mit geringer Wasserlöslichkeit, zum Beispiel ein Arzneimittel, welches mehr als 30 Teile Wasser zur Auflösung von einem Teil des Arzneimittels benötigt, durch Zusammenmischen
A) einer Zusammensetzung, welche sich in einem ersten Behältnis befindet, umfassend das Arzneimittel gelöst in
i) zumindest einem wassermischbaren Lösungsmittel, oder
alternativ
ii) ein amorphes Pulver oder ein Lyophilisat des Arzneimittels, hergestellt aus einer Lösung des Arzneimittels
mit
B) einer Zusammensetzung, welche sich in einem zweiten Behältnis befindet, umfassend
eine liposomale Dispersion.

2. Das Verfahren nach Anspruch 1, worin das zweite Behältnis eine Liposomendispersion beinhaltet, welche optisch klar ist und mindestens eine 40-prozentige Lichttransmission bei einer Wellenlänge von 660nm und bei der Verwendung einer 1cm-Transmissionszelle oder Cuvette erlaubt.

3. Verfahren nach einem der Ansprüche 1 oder 2, worin das wasserlösliche Lösungsmittel, welches sich in dem ersten Behältnis befindet, aus einer Gruppe ausgewählt ist, welche aus Ethanol, 96-prozentiges Ethanol, reinem Glycerin, Propylenglykol, Ethyllaktat, Polyethylenglykol 300, Polyethylenglykol 400, 1,3 Butandiol, Bernsteinsäurediethylester, Triethylcitrat, Dibutylsebacat, Dimethylacetamid, DMSO, Glycerinformal, Glykofurol (Tetraglykol), Isopropanol, Milchsäurebuthylester, N-Methylpyrrolidon, Solketol, Propylencarbonat, Propylenglykoldiacetat, Tetrahydrofurfurylalkohol, Diethylenglykolmonoethylether und Triacetin besteht.

4. Verfahren nach Anspruch 1 oder 2, worin die liposomale Dispersion, welche sich in dem zweiten Behältnis befindet, Liposomen umfasst, welche eine durchschnittliche Partikelgrösse haben, welche kleiner als 1000nm ist.

5. Verfahren nach Anspruch 4, worin die liposomale Dispersion, welche sich in dem zweiten Behälter befindet, eine durchschnittliche Partikelgrösse besitzt, welche kleiner als 300nm ist.

6. Verfahren nach Anspruch 1, worin sich in dem ersten Behältnis zumindest ein Hilfsstoff befindet, ausgewählt aus der Gruppe bestehend aus Mono- und Diacylmembranlipiden wie Ei-Phosphatidylcholin, Soya-Phosphatidylcholin oder Soya-Phosphatidylglycerin, Fettsäuren und deren Salze, Polysorbat 80, Poloxamer und Cremophor® EL.

7. Verfahren nach Anspruch 1, worin die liposomale Dispersion, welche sich in dem zweiten Behältnis befindet, Phospholipide umfasst, welche aus der Gruppe ausgewählt sind, welche aus Phosphatidylcholin, Phosphatidylethanolamin, Sphingomyelin, Phosphatidsäure, Phosphatidyl Inositol, Phosphatidylserin und Phosphatidylglycerin, Glykollipide, Ganglioside und Cerebroside besteht.

8. Verfahren nach Anspruch 6 oder 7, worin das Phospholipid die Formel 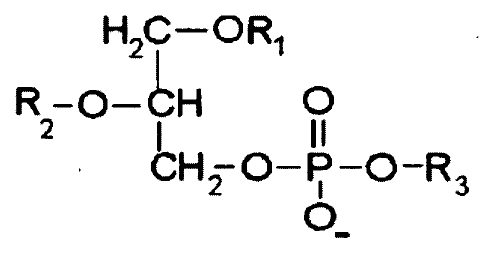
besitzt, worin R1 ein C10-C20 Acyl darstellt, R2 ein Hydrogen oder C10-C20 Acyl darstellt und R3 ein Hydrogen, 2-Trimethylamino-1-Ethyl, 2-Amino-1-Ethyl, C1-C4 Alkyl, C1-C5 Alkyl substituiert durch eine Carboxygruppe, C2-C5 Alkyl substituiert durch eine Carboxy- und Hydroxylgruppe, C2-C5 Alkyl substituiert durch eine Carboxy- und Aminogruppe, eine Inositolgruppe oder eine Glycerylgruppe oder ein Salz derjenigen darstellt.

9. Verfahren nach einem der vorigen Ansprüche, worin die Inhalte des ersten und des zweiten Behältnisses vor Ort gemischt werden, kurz vor der parenteralen Anwendung, wodurch sich eine Zusammensetzung ergibt, welche optisch klar und frei von Ausfällungen oder nicht aufgelöstem Arzneimittel und bereit für die sofortige parenterale Verabreichung ist.

10. Verfahren nach Anspruch 9, worin die Behältnisse einer Gruppe zugehören, welche aus Ampullen, Ampullen mit Gummiverschluss und -kappe, Einkammer- und Zweikammerspritzen, Infusionsbeutel oder -flaschen, geeignet für die parenterale Verabreichung, bestehen.

11. Verfahren nach einem der Ansprüche 1-10, worin das amorphe Pulver, welches sich in dem ersten Behältnis befindet, durch Ausfällung und/oder Lyophilisation aus einer Lösung des Arzneimittels in einem Lösungsmittel hergestellt ist.

12. Ausrüstungsset für die Auflösung eines Arzneimittels mit geringer Wasserlöslichkeit, umfassend
i) ein erstes Behältnis, umfassend
a) ein Arzneimittel mit einer geringen Wasserlöslichkeit, zum Beispiel welche mehr als 30 Teile Wasser benötigt, damit ein Teil des Arzneimittels aufgelöst wird, aufgelöst in
b) zumindest einem wassermischbaren Lösungsmittel, oder alternativ
c) ein amorphes Pulver oder Lyophilisat des Arzneimittels, hergestellt aus einer Lösung des Arzneimittels und optional
d) zumindest ein Tensid ausgewählt aus der Gruppe bestehend aus Phospholipiden, Fettsäuren und deren Salze, Pilysorbate 80, Poloxamer und Cremophor® EL, und
ii) ein zweites Behältnis, umfassend eine liposomale Dispersion;
wobei das Ausrüstungsset vorgesehen ist für das Mischen der Inhalte der beiden Behältnisse, um eine Verabreichungsform des Arzneimittels zu erhalten, geeignet für parenterale, orale, pulmonale und topische Anwendung an einem lebenden Organismus.

13. Ausrüstungsset nach Anspruch 10, worin die Inhalte des ersten und zweiten Behältnisses aseptisch in sterile Ampullen gefüllt sind oder abschliessend sterilisiert sind.

14. Verwendung des Verfahrens aus Anspruch 1 in in-vitro-Zellstudien.

## Revendications

1. Procédé pour solubiliser une substance médicamenteuse avec une faible solubilité dans l'eau, c'est-à-dire une substance médicamenteuse qui requiert plus de 30 parties d'eau pour dissoudre 1 partie de la substance médicamenteuse comprenant le mélange
A) d'un composé qui est contenu dans un premier récipient comprenant ladite substance médicamenteuse dissoute dans
i) au moins un solvant miscible à l'eau ou,
en variante,
ii) une poudre amorphe ou un lyophilisat de la substance médicamenteuse préparé à partir d'une solution de ladite substance médicamenteuse
avec
B) un composé qui est conservé dans un second récipient comprenant
une dispersion liposomale.

2. Procédé selon la revendication 1 dans lequel le second récipient comprend une dispersion liposomale qui est optiquement claire permettant la transmission d'au moins 40% de la lumière à une longueur d'onde de 660 nm en utilisant une cellule ou une cuvette de transmission.

3. Procédé selon la revendication 1 ou 2 dans lequel le solvant miscible à l'eau dans le premier récipient est sélectionné dans le groupe comprenant l'éthanol, l'éthanol à 96%, la glycérine absolue, le propylène glycol, le lactate d'éthyle, le polyéthylène glycol 300, le polyéthylène glycol 400, le 1,3 butanédiol, le diéthylester de l'acide succinique, le citrate de triéthyle, le sébacate de dibutyle, le diméthylacétamide, le diméthylsulfoxyde, le glycérol formal, le glycofurol (tétraglycol), l'isopropanol, le butylester d'acide lactique, le N-méthylpyrrolidone, le Solketol, le propylènecarbonate, le dipropylène glycol acétate, l'alcool de tétrahydrofurfuryle, le monoéthyléther de diéthylène glycol et la triacétine.

4. Procédé selon la revendication 1 ou 2 dans lequel la dispersion liposomale dans le second récipient comprend des liposomes ayant une grandeur moyenne de particules qui est inférieure à 1000 nm.

5. Procédé selon la revendication 4 dans lequel la dispersion liposomale dans le second récipient comprend des liposomes ayant une grandeur moyenne de particules qui est inférieure à 300 nm.

6. Procédé selon la revendication 1 dans lequel le premier récipient comprend au moins un excipient sélectionné dans le groupe comprenant les lipides monoacyles et diacyles à membrane tels que la phosphatidylcholine de l'oeuf, la phosphatidylcholine du soja ou le phosphatidylglycérol du soja, les acides gras et leurs sels, le polysorbate 80, le poloxamer et le Cremophor® EL.

7. Procédé selon la revendication 1 dans lequel la dispersion liposomale dans le second récipient comprend des phospholipides sélectionnés dans le groupe comprenant la phosphatidylcholine, la phosphatidyléthanolamine, la sphingomyéline, l'acide phosphatidique, le phosphatidylinositol, la phosphatidylsérine et le phosphatidylglycérol, les glycolipides, les gangliosides et les cérébrosides.

8. Procédé selon la revendication 6 ou 7 dans lequel le phospholipide est de la formule 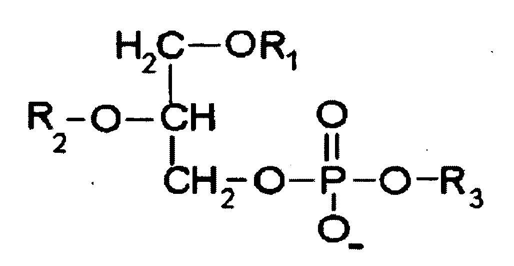
dans lequel R1 représente un acyle en C10-C20, R2 représente l'hydrogène ou un acyle en C10-C20, R3 représente de l'hydrogène, le 2-triméthylamino-1-éthyle, le 2-amino-1-éthyle, un alkyle en C1-C4, un alkyle en C1-C5 substitué par un carboxy, un alkyle en C2-C5 substitué par un carboxy et un hydroxy, un alkyle en C2-C5 substitué par un groupe carboxy et un amino, un groupe inositol ou un groupe glycérol ou un sel de ceux-ci.

9. Procédé selon l'une quelconque des revendications précédentes dans lequel les contenus du premier et du second récipient sont mélangés l'un à l'autre sur place juste avant l'administration parentérale en obtenant ainsi un composé étant optiquement clair et exempt de précipités ou de substance médicamenteuse non associée et prêts pour une administration parentérale immédiate.

10. Procédé selon la revendication 9 dans lequel les récipients sont sélectionnés dans le groupe comportant les ampoules, les flacons avec bouchon de caoutchouc et capuchon, seringue à compartiment simple et double, poche ou flacon pour perfusion, appropriés pour une administration parentérale.

11. Procédé selon l'une quelconque des revendications 1 à 10 dans lequel la poudre amorphe contenue dans le premier récipient est préparée par précipitation et/ou lyophilisation d'une solution de ladite substance médicamenteuse dans un solvant.

12. Kit pour solubiliser une substance médicamenteuse avec une faible solubilité dans l'eau comprenant
i) un premier récipient comprenant
a) une substance médicamenteuse à faible solubilité dans l'eau, c'est-à-dire qui requiert plus de 30 parties d'eau pour dissoudre 1 partie de la substance médicamenteuse, dissoute dans
b) au moins un solvant miscible à l'eau ou en variante
c) une poudre amorphe ou un lyophilisat de la substance médicamenteuse préparé à partir d'une solution de ladite substance médicamenteuse et en option
d) un agent tensio-actif sélecionné dans le groupe comprenant les phospholipides, les acides gras et les sels de ceux-ci, le polysorbate 80, le poloxamer et le Cremophor® EL et
ii) un second récipient comprenant une dispersion liposomale,
ledit kit étant conçu pour mélanger les contenus des deux récipients pour obtenir une substance médicamenteuse avec une forme d'administration appropriée pour l'application orale, pulmonaire et topique à un organisme humain.

13. Kit selon la revendication 10 dans lequel les contenus du premier et du second récipients sont remplis de manière aseptique dans des flacons stériles ou à stérilisation terminale.

14. Utilisation du procédé de la revendication 1 dans des études de cellule in-vitro.
